# EUROPÄISCHE PATENTSCHRIFT

(11) **EP 0 776 877 B1**
(45) Veröffentlichungstag und Bekanntmachung des Hinweises auf die Patenterteilung: **01.12.1999**
(21) Anmeldenummer: 96118555.0
(22) Anmeldetag: 20.11.1996
(51) Int. Cl.: C07C 17/093, C07C 25/13

(54) **Verfahren zur Herstellung von 1-Brom-3,5-difluorbenzol**
Process for the preparation of 1-bromo-3,5-difluorobenzene
Procédé pour la préparation de 1-bromo-3,5-difluorobenzène

(30) Priorität: 01.12.1995 DE 19544870
(43) Veröffentlichungstag der Anmeldung: 04.06.1997
(73) Patentinhaber: Clariant GmbH, 65929 Frankfurt am Main (DE)
(72) Erfinder: Neuber, Marita, Dr., 60528 Frankfurt (DE); Schach, Thomas, Dr., 64579 Gernsheim (DE); Papenfuhs, Theodor, Dr., 60433 Frankfurt (DE); Pfirmann, Ralf, Dr., 64347 Griesheim (DE); Roscher, Günter, Dr., 65779 Kelkheim (DE)

(56) Entgegenhaltungen:
- EP-A- 0 596 684
- PATENT ABSTRACTS OF JAPAN vol. 008, no. 041 (C-211), 22.Februar 1984 & JP 58 201734 A (CHISSO KK), 24.November 1983,

## Beschreibung

Die vorliegende Erfindung betrifft ein Verfahren zur Herstellung von 1-Brom-3,5-difluorbenzol.

1-Brom-3,5-difluorbenzol ist ein Vorprodukt für die Herstellung von Pharmazeutika und von Flüssigkristallen.

Nach dem Stand der Technik kann 1-Brom-3,5-difluorbenzol durch folgende Verfahren hergestellt werden:
1. durch Bromierung von 2,4-Difluoranilin, anschließende Diazotierung mit NaNO₂ und Deaminierung durch unterphosphorige Säure in saurer Lösung.
   A. Roe und W. F. Little, J. Org. Chem. 20 (1955) 1577/1590 erhalten nach diesem Verfahren 1-Brom-3,5-difluorbenzol in einer Ausbeute von 60 %.
   L. I. Kruse et al., J. Org. Med., 29 (1986) 887/889 und J. Org. Med., 30 (1987) 486/494 erzielen eine Gesamtausbeute an 1-Brom-3,5-difluorbenzol von 57 %.
   In US 5 157 169 ist eine Ausbeute an 1-Brom-3,5-difluorbenzol von 70 % beschrieben.

   Die nach diesem Verfahren erzielten Ausbeuten sind insbesondere beim Verfahrensschritt der Deaminierung gering, teure Ausgangsmaterialien gehen verloren. Das Verfahren ist vielstufig.
2. durch photochemische Bromierung von m-Difluorbenzol
   R. Bolton und E. S. E. Owen, J. Fluor. Chem. 46 (1990) 393/406 beschreiben, daß nach diesem Verfahren 19 % 1-Brom-3,5-difluorbenzol neben 10 % der anderen Bromdifluorbenzol-Isomeren und 43 % an höherbromierten Difluorbenzolen entstehen. Bei diesem Verfahren ist die Bildung von 1-Brom-3,5-difluorbenzol wenig selektiv.

   Durch direkte Bromierung von m-Difluorbenzol ist 1-Brom-3,5-difluorbenzol nicht erhältlich. Dabei entsteht selektiv 1-Brom-2,4-difluorbenzol.
3. durch Isomerisierung von 1-Brom-2,4-difluorbenzol
   In EP 63066 wird die Isomerisierung von 1-Brom-2,4-difluorbenzol in Gegenwart von Alkalibasen, bevorzugt Alkaliamiden, und makrocyclischen Verbindungen wie z.B. Polyethern durchgeführt.
   Dieses Verfahren weist eine Reihe von Nachteilen auf. Die makrocyclischen Verbindungen haben eine komplizierte Struktur und sind daher sehr teuer. Sie werden nach der Reaktion nicht zurückgewonnen. Zur Produktaufarbeitung muß das basische Reaktionsgemisch neutralisiert werden, wobei Salze anfallen. Wegen der geringen Ausbeuten an 1-Brom-3,5-difluorbenzol und 63 % und der hohen Materialkosten ist dieses Verfahren wenig wirtschaftlich.
   In JP 04 182 440 (CA 117: 170951n) wird die Isomerisierung von 1-Brom-2,4-difluorbenzol in Gegenwart von Alkalimetall-t-butoxiden oder Aluminiumhalogeniden beschrieben. Auch bei diesem Verfahren ist ein zusätzlicher, salzbildender Neutralisiationsschritt erforderlich. Die Ausbeute an 1-Brom-3,5-difluorbenzol beträgt nur 41,4 %.
   In EP 648 724 wird die Isomerisierung von 1-Brom-2,4-dilfuorbenzol mit sauren Zeolithen als Katalysatoren beschrieben. Nachteilig bei diesem Verfahren sind zum einen große Mengen, die im Kreis gefahren werden müssen, da der Umsatz beschränkt ist, zum anderen der hohe apparative Aufwand für eine kontinuierliche Apparatur.

Andere Einsatzmaterialien sind für die Herstellung von 1-Brom-3,5-difluorbenzol in der Literatur nicht beschrieben.

Es bestand somit ein Bedarf nach einem Verfahren, das die beschriebenen Nachteile vermeidet und 1-Brom-3,5-difluorbenzol in hohen Ausbeuten und hoher Selektivität technisch einfach zugänglich macht.

Diese Aufgabe wird gelöst durch ein Verfahren zur Herstellung von 1-Brom-3,5-difluorbenzol, dadurch gekennzeichnet, daß 3,5-Difluoranilin zum Diazoniumsalz und dieses anschließend in Gegenwart von CuBr und HBr zu 1-Brom-3,5-difluorbenzol umgesetzt wird.

3,5-Difluoranilin ist eine bekannte Verbindung. Ein vorteilhafter Weg zur Herstellung von 3,5-Difluoranilin ist z.B. in EP 562 435 beschrieben.

Die Herstellung von halogenierten Aromaten aus den entsprechenden Anilinen ist bekannt und in der Literatur ausführlich beschrieben (Sandmeyer-Reaktion). So finden sich Synthesevorschriften für die Diazotierung von Anilinen sowie die Sandmeyer-Reaktion der Diazonium-Salze zu den entsprechenden halogenierten Aromaten, z.B. in Organikum, 4. Auflage (1964), S. 491/492 und S. 497/499, Org. Synth., Coll. Vol. 3, S. 185-187, Houben-Weyl, Halogenverbindungen, Band V/4 (1960), S. 438/446. Nach diesen Vorschriften lassen sich halogenierte Aromaten in hohen Ausbeuten zwischen 80 und 95 % aus den entsprechenden Anilinen herstellen.

Umso auffälliger ist es jedoch, daß in der Fachliteratur die Umsetzung von 3,5-Difluoranilin zu 1-Brom-3,5-difluor-benzol nicht erwähnt ist.

Dagegen wird in mehreren Arbeiten die Herstellung von 1-Brom-3,5-difluorbenzol ausgehend von 2,4-Difluoranilin, einem Isomeren des 3,5-Difluoranilin, dargestellt. 2,4-Difluoranilin wird durch Bromierung zuerst zum 2-Brom-4,6-difluoranilin umgesetzt, anschließend wird mit NaNO₂ diazotiert und mit unterphosphoriger Säure in saurer Lösung zu 1-Brom-3,5-difluorbenzol deaminiert. Dieser Weg beinhaltet eine Stufe mehr als das erfindungsgemäße Verfahren. Darüber hinaus sind die Ausbeuten der Deaminierung mit unterphosphoriger Säure wesentlich geringer als die in der Literatur beschriebenen Ausbeuten für die Sandmeyer-Reaktion.

Da die direkte Umsetzung von 3,5-Difluoranilin zu 1-Brom-3,5-difluorbenzol nicht beschrieben ist, liegt es nahe anzunehmen, daß diese Reaktion nur mit Schwierigkeiten durchzuführen ist und der Umweg über 2,4-Difluoranilin, Bromierung, Diazotierung und Deaminierung günstiger ist.

Dagegen wurde nun überraschenderweise gefunden, daß 1-Brom-3,5-difluorbenzol mit hoher Ausbeute direkt aus 3,5-Difluoranilin durch Diazotierung und anschließender Sandmeyer-Reaktion hergestellt werden kann.

Für die Diazotierung von 3,5-Difluoranilin mit äquimolarer Menge NaNO₂ ist ein 2,5 bis 3-facher Überschuß an HBr ausreichend. Es hat sich bewährt, die Säure 48 %ig zuzugeben und die Reaktion bei tiefen Temperaturen, am günstigsten bei Temperaturen <10°C durchzuführen.

NaNO₂ wird als etwa 50 %ige Lösung zugegeben. Durch Überprüfung der Farbreaktion mit Jodstärkepapier wird sichergestellt, daß kein Überschuß an Nitrit vorhanden ist. Gegebenenfalls wird etwas Harnstoff zugegeben.

Das gebildete Diazoniumsalz wird bis zur Weiterverarbeitung auf etwa 10°C gekühlt. Das gesamte Diazoniumsalz kann entweder bei 0°C zu der CuBr/HBr gegeben werden und erst anschließend wird zum Sieden erhitzt. Oder aber die vorgelegte CuBr/HBr-Mischung wird zum Sieden erhitzt und das gekühlte Diazoniumsalz wird portionsweise zugegeben, so daß die gesamte Mischung am Sieden bleibt.

Das gebildete 1-Brom-3,5-difluorbenzol destilliert zusammen mit Wasserdampf ab. Es wird erhitzt, bis keine Gasentwicklung mehr auftritt. Anschließend wird, um alle Reaktionsprodukte zu gewinnen, mit Hilfe von Wasserdampf das restliche organische Produkt überdestilliert.

Die organische Phase wird von der Wasserphase abgetrennt, mit verdünnter Lauge neutralisiert, gewaschen, getrocknet und anschließend reindestilliert.

Im folgenden ist in einem Beispiel das erfindungsgemäße Verfahren veranschaulicht.

### Beispiel 1:

### Diazotierung:

In einem 500 ml Vierhalskolben mit Rührer, Thermometer und Eintropfer werden 64,5 g (0,5 mol) 3,5-Difluoranilin und 210 ml 48 %ige Hbr (1,25 mol Hbr) vorgelegt, bei <10°C 34,5 g (0,5 mol) NaNO₂ in 60 ml Wasser unter Rühren zugegeben. Es wird so lange NaNO₂ zugegeben, bis sich gerade freie HNO₂ durch Farbreaktion von Jodstärke-Papier nachweisen läßt.

### Sandmeyer-Reaktion:

Die bei 10°C gehaltene Reaktionsmischung wird portionsweise unter Rühren zu einem siedenden Gemisch aus 35,4 g (0,25 mol) CuBr in 46 ml 48 %iger Hbr (0,27 mol Hbr) in einem 1 l Vierhalskolben mit Destillationsbrücke, Dampfeinleitungsrohr, Tropfrichter und Thermometer gegeben. Es wird erhitzt, so daß das Gemisch weiter siedet. Organisches Produkt und Wasser destillieren schon während der Zugabe von Diazoniumsalz ab. Nach beendeter Zugabe wird mit Dampf so lange weiterdestilliert, bis kein organisches Produkt in der Vorlage mehr anfällt.

Die Phasen in der Vorlage werden getrennt, die organische Phase wird zunächst mit Wasser gewaschen, dann mit 5 %iger NaOH neutralisiert und anschließend neutralgewaschen. Nach Trocknen über Na₂SO₄ und Reindestillation werden 80 g (0,41 mol) 1-Brom-3,5-difluorbenzol erhalten, was einer Ausbeute von 83 % der Theorie entspricht.

## Patentansprüche

1. Verfahren zur Herstellung von 1-Brom-3,5-difluorbenzol, dadurch gekennzeichnet, daß 3,5-Difluoranilin zum Diazoniumsalz und dieses anschließend in Gegenwart von CuBr mit HBr umgesetzt wird.

2. Verfahren nach Anspruch 1, dadurch gekennzeichnet, daß 3,5-Difluoranilin mit NaNO₂ in Gegenwart von HBr diazotiert wird.

3. Verfahren nach mindestens einem der Ansprüche 1 oder 2, dadurch gekennzeichnet, daß bei der Diazotierung die 2,5- bis 3-fache molare Menge an HBr bezogen auf 3,5-Difluoranilin eingesetzt wird.

4. Verfahren nach mindestens einem der Ansprüche 1 bis 3, dadurch gekennzeichnet, daß für die Diazotierung 48 %ige HBr eingesetzt wird.

5. Verfahren nach mindestens einem der Ansprüche 1 bis 4, dadurch gekennzeichnet, daß in der 2. Reaktionsstufe das Diazoniumsalz portionsweise zu einem siedenden Gemisch aus CuBr und HBr gegeben wird.

6. Verfahren nach mindestens einem der Ansprüche 1 bis 5, dadurch gekennzeichnet, daß 0,2 bis 0,5 mol CuBr pro mol 3,5-Difluoranilin verwendet werden.

7. Verfahren nach mindestens einem der Ansprüche 1 bis 5, dadurch gekennzeichnet, daß für die 2. Reaktionsstufe 0,2 bis 0,5 mol HBr pro mol 3,5-Difluoranilin verwendet werden.

## Claims

1. A process for preparing 1-bromo-3,5-difluorobenzene, which comprises converting 3,5-difluoroaniline into its diazonium salt and then reacting the diazonium salt with HBr in the presence of CuBr.

2. The process as claimed in claim 1, wherein 3,5-difluoroaniline is diazotized with NaNO₂ in the presence of HBr.

3. The process as claimed in at least one of claims 1 or 2, wherein the diazotization is carried out with 2.5 to 3 times the molar amount of HBr based on 3,5-difluoroaniline.

4. The process as claimed in at least one of claims 1 to 3, wherein the diazotization is carried out with 48% strength HBr.

5. The process as claimed in at least one of claims 1 to 4, wherein, in the 2nd reaction step, the diazonium salt is added a little at a time to a boiling mixture of CuBr and HBr.

6. The process as claimed in at least one of claims 1 to 5, wherein from 0.2 to 0.5 mol of CuBr is used per mole of 3,5-difluoroaniline.

7. The process as claimed in at least one of claims 1 to 5, wherein, for the 2nd reaction step, from 0.2 to 0.5 mol of HBr is used per mole of 3,5-difluoroaniline.

## Revendications

1. Procédé de préparation de 1-bromo-3,5-difluorobenzène, caractérisé en ce que l'on convertit de la 3,5-difluoroaniline en sel de diazonium et ensuite on fait réagir ce dernier avec du HBr en présence de CuBr.

2. Procédé selon la revendication 1, caractérisé en ce que l'on diazote la 3,5-difluoroaniline avec du NaNO₂ en présence de HBr.

3. Procédé selon l'une au moins des revendications 1 ou 2, caractérisé en ce que, lors de la diazotation, on utilise une quantité molaire de HBr 2,5 à 3 fois supérieure à la quantité de 3,5-difluoroaniline.

4. Procédé selon l'une au moins des revendications 1 à 3, caractérisé en ce que l'on utilise pour la diazotation du HBr à 48 %.

5. Procédé selon l'une au moins des revendications 1 à 4, caractérisé en ce que, dans la deuxième étape de la réaction, on ajoute le sel de diazonium à un mélange de CuBr et de HBr en ébullition.

6. Procédé selon l'une au moins des revendications 1 à 5, caractérisé en ce que l'on utilise de 0,2 à 0,5 mol de CuBr par mol de 3,5-difluoroaniline.

7. Procédé selon l'une au moins des revendications 1 à 5, caractérisé en ce que, pour la deuxième étape de la réaction, on utilise de 0,2 à 0,5 mol de HBr par mol de 3,5-difluoroaniline.
